(19) Europäisches Patentamt
European Patent Office
Office européen des brevets

(11) Publication number: 0 020 065
B1

(12) # EUROPEAN PATENT SPECIFICATION

(45) Date of publication of patent specification: 23.02.83

(51) Int. Cl.³: C 07 C 57/075, C 07 C 69/54, C 07 C 51/50, C 07 C 67/62

(21) Application number: 80301625.2

(22) Date of filing: 19.05.80

(54) **A method for inhibiting the polymerization of acrylic monomers in aqueous liquids containing them.**

(30) Priority: 21.05.79 US 41035

(43) Date of publication of application: 10.12.80 Bulletin 80/25

(45) Publication of the grant of the patent: 23.02.83 Bulletin 83/8

(84) Designated Contracting States: DE FR GB

(56) References cited: DE - A - 2 856 823

(73) Proprietor: Rohm and Haas Company
Independence Mall West
Philadelphia, Pennsylvania 19105 (US)

(72) Inventor: Backus, Andrew Chase
295 Walden Street
Cambridge, Massachusetts, 02138 (US)
Inventor: Stewart, Thomas
Gayman Road, R.D.1
Doylestown, Pennsylvania, 18901 (US)

(74) Representative: Wood, Peter Worsley Spencer et al,
Rohm and Haas Company Patent Department
Chesterfield House Barter Street
London, WC1A 2TP (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 020 065 B1

# A method for inhibiting the polymerization of acrylic monomers in aqueous liquids containing them

This invention concerns a method of inhibiting the polymerization of an aqueous liquid containing an acrylic monomer by adding to the liquid a salt of a nitrosophenol.

It is well known in the art to employ inhibitors to prevent or reduce unwanted polymerization. For example British Patent No. 1,064,845 discloses inhibition of free radical polymerization of styrene, and various acrylates and methacrylates employing nitrosobenzene, *p*-nitrosophenol, *beta*-nitroso-*alpha*-naphthol and N-nitrosodiphenylamines. Also U.K. Patent No. 1,477,298 discloses the inhibition of polymerization of acrylic acid with an aromatic nitrous compound containing an NO-group bound directly to a carbon atom of the aromatic nucleus such as alpha-nitroso-beta-naphthol.

However, the search continues for a polymerization inhibitor which can be employed in the process for preparing methacrylic acid, acyrlic acid and esters and amides thereof which inhibitor, particularly when used as the sole inhibitor, will prevent polymerization of these materials in aqueous by-products streams, recycle streams and waste streams containing these vinyl monomers.

It is the practice of those in the industry to employ different inhibitors or mixtures of inhibitors at various stages of the process. For example, the use of methylene blue chloride and/or hydroquinone are two well known water-soluble inhibitors which have been employed but which do not completely prevent spontaneous polymerization of the vinyl monomers. This spontaneous polymerization not only causes the reduction in yield of the final product but also results in equipment failure.

In our copending French Patent Application No. PV 79 00229 (Publication No. 2414036), West German Patent Application No. P 2856823.2 (Publication No. 2856823) and U.K. Patent Application No. 26124/78 (Publication No. 1601979), we have described and claimed the use of salts of various nitrosophenols to inhibit the polymerization of acrylate or methacrylate salts. We have now found that similar salts of nitrosophenols, particularly when used without any other inhibitor, can advantageously be used to inhibit the polymerization, in aqueous liquids, of acrylic monomers which are other than, and are free of, salts of acrylic or methacyrlic acid.

In accordance with the invention, there is provided a method of inhibiting the polymerization of acrylic monomers in an aqueous liquid which contains at least one monomer selected from acrylic acid, acrylic acid esters, acrylamide (in free amide or salt form), methacrylic acid, methacrylic acid esters and methacrylamide (in free acid or salt form) but which aqueous liquid has a pH below 6 and contains no salt of acrylic or methacrylic acid, which method is characterized by adding to said aqueous liquid a polymerization inhibiting amount of a soluble alkali metal, alkaline earth metal, ammonium or alkyl-substituted ammonium salt of nitrosophenol, nitrosonaphthol or nitrosoquinolinol each of the last three nitrosophenols being optionally substituted with one or two of the same or different substituents selected from halo, alkyl, alkoxy and $-SO_3H$.

In addition to the cost advantage of being able to employ one inhibitor, the amount of inhibitor previously needed has also been reduced by the invention thereby further improving the cost advantage and also improving environmental aspects.

The salts used are much more stable and therefore safer to use than the free phenols. For example, free nitrosophenols have been known to explode on impact or to ignite spontaneously; the salts do not. The free nitrosophenols are only sparingly soluble in water whereas the salts are much more soluble and provide an excellent means to deliver the nitrosophenols into the aqueous liquid containing the acrylic monomer.

The nitrosophenol salts used may be represented by the formula

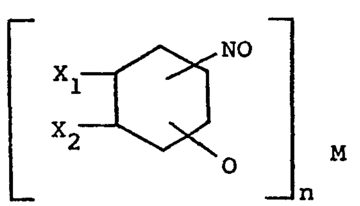

(I)

wherein $X_1$ and $X_2$ are hydrogen or are joined together to form a benzene or pyridine ring, M is an alkali metal, an alkaline earth metal, ammonium or alkyl-substituted ammonium and n is 1 or 2 depending on the valency of M. There may also be used compounds of Formula I containing up to 2 of the same or different ring substitutents selected from halo (e.g. chloro, fluoro or bromo), alkyl (particularly $(C_1-C_5)$alkyl such as methyl, ethyl, n-propyl, n-butyl or n-pentyl) and alkoxy (particularly $(C_1-C_5)$alkoxy such as methoxy, ethoxy, n-propoxy, n-butoxy or n-pentyloxy).

M, in Formula I above typically represents lithium, sodium, potassium, calcium, barium, ammonium or alkyl-substituted ammonium such as ammonium substituted with up to 4 $(C_1-C_{16})$alkyl groups. Representative examples of substituted ammonium groups are tetramethylammonium, diethylammonium and cetylammonium.

When it is desired to inhibit the polymerization of esters, the usual esters will be $(C_1-C_5)$alkyl esters notably methyl, ethyl, n-butyl, n-propyl and n-pentyl esters.

The aqueous liquids to which the inhibitor salts are added may be those containing ester, unreacted alcohol (e.g. methyl, ethyl or n-butyl alcohol), unreacted (meth)-acrylic acid and water usually in amounts from 1% to 98% based on the weight of the liquid.

In the salt inhibitors that are used, it is preferred to have the nitroso groups ortho or para to the hydroxy salt radical.

Specific examples of salt inhibitors are salts of 4-nitroso-2-chlorophenol, 5-methoxy-2-nitrosophenol, 8-hydroxy-5-nitrosoquinoline, 6-chloro-4-nitroso-2-methylphenol, 4-nitrosophenol, 1-nitroso-2-naphthol and 1-nitroso-2-naphthol-3,6-disulfonic acid. The most preferred salt is the sodium salt of 4-nitrosophenol.

The salts of the nitrosophenols may be employed in an amount in the range of from about 10 parts per million to about 5,000 parts per million based on the weight of the aqueous liquid containing the acrylic monomer. The preferred amount is in the range of from about 100 to about 500 parts per million.

The pH of the aqueous liquid containing the acrylic monomer system will naturally vary but it is always below 6, the preferred pH is in the range of from 2 or 4 to <6.

The following examples, which include comparative data and in which parts and percentages are on a weight basis, are presented to illustrate.

Examples 1 to 5 below show a comparison of the use of sodium *p*-nitrosophenolate with methylene blue and a control (no inhibitor) employed at various stages of a process for preparing methyl methacrylate from methacrylic acid and methanol. Both sodium *para*-nitrosophenolate (NapNP) and methylene blue (MB) were employed at the rate of 50 parts per million based on total sample stream weight. The sample streams were held at 55°C and checked daily for polymer formation over 16 days. The following table shows the results.

| | | Composition | | | | | | Days to Polymerization | | |
|---|---|---|---|---|---|---|---|---|---|---|
| Ex No | Stream Name | Methanol | Methyl Methacrylate | Methacrylic Acid | Acetone | Water | pH | Control | MB | NaPNP |
| 1 | Ester separator upper layer | 5 | 78 | 13 | 1 | 3 | 3.8 | 5 | 12 | >16 |
| 2 | Wash overhead | 1.1 | 97 | 0.2 | 0.5 | 1.2 | 2.8 | 3 | 7 | >16 |
| 3 | Light ends column bottoms | 11 | 70 | 0.5 | 0.5 | 18 | 3.8 | 3 | 4 | >16 |
| 4 | Acid stripper overhead | 41.5 | 2.5 | 3.0 | 1.0 | 52 | 2.5 | 3 | 4 | >16 |
| 5 | Flash column condenser lower layer | 52 | 20.5 | 2.0 | 0.5 | 25 | 3.5 | 3 | 5 | >16 |

### Example 6

*Use of sodium p-nitrosophenolate in the production of n-butyl acrylate*

On day 1 an aqueous solution of 0.3% methylene blue chloride and 0.3% sodium *p*-nitrosophenolate was inserted into the aqueous inhibitor feed leading to 18 aqueous inhibitor injection points in the process. No polymer difficulties were detected. On day 5 the use of only sodium *p*-nitrosophenolate was begun employing 0.3% aqueous solution. For a period of 22 days the process was continued employing only sodium *p*-nitrosophenolate. Again no polymer was observed. During the course of production the inhibitor solution feed rate to the ester main condenser (i.e. the condenser in the overhead system from the esterification reactor and which leads to the ester separator aqueous layer) was reduced to 35% of the rate previously employed using methylene blue. Ester separator aqueous layer during butyl acrylate production had the following composition: 97.05% water; 2.60% n-butanol; 0.25% acrylic acid and 0.10% n-butyl acrylate and had a pH in the range of from 4 to 5. Analysis of two samples taken during the run showed a concentration of 63 and 27 parts per million of inhibitor. Acidification loop separator aqueous layer, another stream receiving the aqueous inhibitor, had the following composition: 25% ammonium sulfate; 0.8% n-butanol; 1.4% acrylic acid; 0.2% butyl acrylate; 71.1% water and 1.5% sulfuric acid and had a pH in the range of from 2 to 2.5. The acidification loop separator aqueous layer arose on acidification with sulfuric acid of an aqueous ammonium acrylate stream followed by extraction of the bulk of the liberated acrylic acid with an organic solvent.

### Example 7

*Use of sodium p-nitrosophenolate as the inhibitor in the production of ethyl acrylate*

During the production of ethyl acrylate, sodium *p*-nitrosophenolate was employed as the sole aqueous phase inhibitor reducing the rate of inhibitor feed by 62% of the rate previously used when methylene blue chloride was employed. No aqueous phase polymer problems occurred. One stream receiving the inhibitor was the ester separator aqueous layer having the following composition: 75.95% water; 21% ethanol; 0.05% acrylic acid and 3.00% ethyl acrylate having a pH in the range of 5 to 6.

### Example 8

*Acrylic acid*

25.6 g of uninhibited glacial acrylic acid and 5.2 g of concentrated sulfuric acid were dissolved in 75.0 g of de-ionized water to give an aqueous solution having a pH of 0.9. The solution was divided in half. One portion was treated with 195 ppm of sodium *p*-nitrosophenolate (based on the solution weight) and the other left untreated. Both were held at 55°C under an atmosphere of nitrogen. Within several hours the untreated sample showed signs of vinyl polymerization; the sample treated with sodium *p*-nitrosophenolate showed no signs of polymerization after a week, when the test was stopped.

**Claims**

1. A method of inhibiting the polymerization of acrylic monomers in an aqueous liquid which contains at least one monomer selected from acrylic acid, acrylic acid esters, acrylamide (in free amide or salt form), methacrylic acid, methacrylic acid esters and methacrylamide (in free acid or salt form) but which aqueous liquid has a pH below 6 and contains no salt or acrylic or methacrylic acid, which method is characterized by adding to said aqueous liquid a polymerization inhibiting amount of a soluble alkali metal, alkaline earth metal, ammonium or alkyl-substituted ammonium salt of nitrosophenol, nitrosonaphthol or nitrosoquinolinol each of the last three nitrosophenols being optionally substituted with one or two of the same or different substituents selected from halo, alkyl, alkoxy and $—SO_3H$.

2. A method as claimed in Claim 1, wherein the nitrosophenol employed, if substituted, is substituted with one or two of the same or different substituents selected from chloro, $(C_1—C_5)$alkyl, $(C_1—C_5)$alkoxy and $—SO_3H$.

3. A method as claimed in Claim 1 or 2, wherein the pH of the aqueous liquid is from 0 to $<6$.

4. A method as claimed in Claim 3, wherein the said pH is from 2 to $<6$.

5. A method as claimed in any one of Claims 1 to 4, wherein the aqueous liquid is one obtained from a process for the esterification of (meth)acrylic acid with methanol, ethanol or n-butanol and contains, as the case may be, (1) methyl, ethyl or n-butyl (meth)acrylate, (2) unreacted (meth)acrylic acid and (3) unreacted methanol, ethanol or n-butanol.

6. A method as claimed in any one of the preceding claims, wherein there is used as inhibitor the sodium salt of 4-nitrosophenol.

7. A method as claimed in Claim 6, wherein the sodium salt of 4-nitrosophenol is used as the sole inhibitor.

**Patentansprüche**

1. Verfahren zur Inhibierung der Polymerisation von Acrylmonomeren in einer wäßrigen Flüssigkeit, die wenigstens ein Monomeres enthält, ausgewählt aus Acrylsäure, Acrylsäureestern, Acrylamid (in freier Amid- oder Salzform), Methacrylsäure, Methacrylsäureestern sowie Methacrylamid (in Form der freien Säure oder in Salzform), wobei jedoch die wäßrige Flüssigkeit einen pH unterhalb 6 aufweist und kein Salz von Acrylsäure oder Methacrylsäure enthält, dadurch gekennzeichnet, daß der wäßrigen Flüssigkeit eine die Polymerisation inhibierende Menge eines löslichen Alkalimetallsalzes, Erdalkalimetallsalzes, Ammoniumsalzes oder alkylsubstituierten Ammoniumsalzes von Nitrosophenol, Nitrosonaphthol oder Nitrosochinolinol zugesetzt wird, wobei jedes der letzten drei Nitrosophenole gegebenenfalls mit einem oder zwei gleichen oder verschiedenen Substituenten substituiert ist, ausgewählt aus Halogen, Alkyl, Alkoxy sowie —$SO_3H$.

2. Verfahren nach Anspruch 1, dadurch gekennzeichnet, daß das eingesetzte Nitrosophenol, falls es substituiert ist, mit einem oder zwei gleichen oder verschiedenen Substituenten substituiert ist, ausgewählt aus Chlor, ($C_1$—$C_5$)Alkyl, ($C_1$—$C_5$)Alkoxy sowie —$SO_3H$.

3. Verfahren nach Anspruch 1 oder 2, dadurch gekennzeichnet, daß der pH der wäßrigen Flüssigkeit zwischen 0 und <6 gehalten wird.

4. Verfahren nach Anspruch 3, dadurch gekennzeichnet, daß ein pH von 2 bis <6 eingehalten wird.

5. Verfahren nach einem der Ansprüche 1 bis 4, dadurch gekennzeichnet, daß die eingesetzte wäßrige Flüssigkeit eine solche ist, die bei einem Verfahren zur Veresterung von (Meth)acrylsäure mit Methanol, Ethanol oder n-Butanol anfällt und je nach der Situation (1) Methyl-, Ethyl- oder n-Butyl (meth)acrylat, (2) nichtumgesetzte (Meth)acrylsäure und (3) nichtumgesetztes Methanol, Ethanol oder n-Butanol enthält.

6. Verfahren nach einem der vorhergehenden Ansprüche, dadurch gekennzeichnet, daß als Inhibitor das Natriumsalz von 4-Nitrosophenol verwendet wird.

7. Verfahren nach Anspruch 6, dadurch gekennzeichnet, daß das Natriumsalz von 4-Nitrosophenol als einziger Inhibitor verwendet wird.

**Revendications**

1. Procédé d'inhibition de la polymérisation de monomères acryliques dans un liquide aqueux qui contient au moins un monomère choisi parmi l'acide acrylique, les esters de l'acide acrylique, l'acrylamide (sous la forme de l'amide libre ou d'un sel), l'acide méthacrylique, les esters de l'acide méthacrylique et le méthacrylamide (sous la forme d'acide libre ou d'un sel), ce liquide aqueux ayant un pH inférieur à 6 et ne contenant pas de sel d'acide acrylique ou méthacrylique, ce procédé étant caractérisé par l'addition audit liquide aqueux d'une quantité inhibant la polymérisation d'un sel soluble de métal alcalin, de métal alcalino-terreux, d'ammonium ou d'ammonium alcoyl-substitué de nitrosophénol, de nitrosonaphtol ou de nitrosoquinolinol, chacun des trois derniers nitrosophénols étant éventuellement substitué par un ou deux substituants semblables ou différents choisis parmi halogéno, alcoyle, alcoxy et —$SO_3H$.

2. Procédé selon la revendication 1, dans lequel le nitrosophénol employé, s'il est substitué, est substitué par un ou deux substituants semblables ou différents choisis parmi chloro, alcoyle en ($C_1$—$C_5$), alcoxy en ($C_1$—$C_5$), —$SO_3H$.

3. Procédé selon la revendication 1 ou 2, dans lequel le pH du liquide aqueux est de 0 à <6.

4. Procédé selon la revendication 3, dans lequel ledit pH est de 2 à <6.

5. Procédé selon l'une quelconque des revendications 1 à 4, dans lequel le liquide aqueux est obtenu à partir d'un procédé d'estérification de l'acide (méth) acrylique avec le méthanol, l'éthanol ou le n-butanol et contient, selon le cas, (1) du (méth) acrylate de méthyle, d'éthyle ou de n-butyle, (2) de l'acide (méth) acrylique n'ayant pas réagi et (3) du méthanol, de l'éthanol ou du n-butanol n'ayant pas réagi.

6. Procédé selon l'une quelconque des revendications précédentes, dans lequel on utilise comme inhibiteur le sel de sodium du 4-nitrosophénol.

7. Procédé selon la revendication 6, dans lequel le sel de sodium du 4-nitrosophénol est utilisé comme seul inhibiteur.